# EUROPÄISCHE PATENTSCHRIFT

(11) **EP 2 987 516 B1**
(45) Veröffentlichungstag und Bekanntmachung des Hinweises auf die Patenterteilung: **10.01.2018**
(21) Anmeldenummer: 15181222.9
(22) Anmeldetag: 17.08.2015
(51) Int. Cl.: A61M 5/00

(54) **MAGAZIN FÜR MINDESTENS EINE SUBKUTANINJEKTIONSNADEL**
CARTRIDGE FOR AT LEAST ONE SUBCUTANEOUS INJECTION NEEDLE
MAGASIN POUR AU MOINS UNE AIGUILLE D'INJECTION SOUS-CUTANEE

(30) Priorität: 19.08.2014 DE 102014111789; 19.08.2014 DE 202014103829 U
(43) Veröffentlichungstag der Anmeldung: 24.02.2016
(73) Patentinhaber: IME-DC GmbH International Medical Equipment - Diabetes Care, 95030 Hof (DE)
(72) Erfinder: Kamalak, Serif, 95032 Hof (DE)
(74) Vertreter: Sperschneider, Alexandra

(56) Entgegenhaltungen:
- EP-A1- 2 522 380
- EP-A2- 2 420 270
- WO-A1-00/54691
- WO-A1-92/12743
- WO-A1-2013/125443
- DE-T2- 60 131 847
- DE-T2- 69 805 584
- GB-A- 2 462 303
- JP-A- 2007 185 499
- US-A- 5 545 145
- US-A1- 2012 037 655
- US-A1- 2013 105 345

## Beschreibung

Die Erfindung betrifft ein Magazin für mindestens eine Subkutaninjektionsnadel, bestehend aus einer Hohlnadel, die in einer Nadelnabe beidseitig vorstehend fixiert ist, wobei das eine Ende mit der Nadelnabe aus der Stirnwand einer auf ein Verbindungsstück einer Spritze oder eines Injektionsgerätes aufschraubbaren oder aufsteckbaren Muffe vorsteht, die das andere Ende der Hohlnadel umgibt, wobei das andere Ende bei der Fixierung der Muffe an dem Verbindungsstück eine Gummimembran, die zumindest einen Teil der Endfläche des Verbindungsstückes bildet, durchstößt, wobei die Subkutaninjektionsnadel in bekannter Weise von einem Aufbewahrungsbehälter umgeben ist, der über eine das überstehende Ende der Hohlnadel schützende Schutzkappe und die Muffe oder über die Nadel und die Muffe greift und mit einer Abdeckung an der offenen Muffenseite steril versiegelt ist.

Aus der DE 601 31 847 T2, Figuren 4a bis 4c, ist ein Magazin in Form eines zylindrischen Gehäuses bekannt, in welchem axial aneinander gereiht mehrere Subkutaninjektionsnadeln eingeführt sind und aus welchen einzelne Nadeln mit einem Verbindungsstück eines Injektionsgerätes bzw. einer Injektionspatrone verbindbar sind. Die Entnahme und die Adaption mit dem Injektionsgerät sind dabei aufwändig. Des Weiteren können die Subkutaninjektionsnadeln im sterilen versiegelten Zustand nicht in das Magazin einzeln eingeschoben werden. Bestenfalls kann das Magazin insgesamt durch eine Abdeckung des zylinderförmigen Körpers im sterilen Zustand verschlossen werden. Bei einmaligem Öffnen ist die Gefahr gegeben, dass die darin befindlichen Subkutaninjektionsnadeln in unerwünschter Weise kontaminiert werden können.

Ebenso ist aus US 5 545 145 ein Magazin mit einem zylindrisch ausgeformten Gehäuse, welches an ein Injektionsgerät geklippt werden kann, bekannt. Durch axiales Hintereinanderreihen verpackter Nadeln im Magazin und Notwendigkeit einer Kappe zum Verschliessen des Magazins bringt jene Formgebung allerdings Nachteile in der Handhabung mit sich. Ferner ist aus der DE 698 05 584 T2 ein Magazin zum Aufbewahren von Injektionsnadeln in Form eines Revolvermagazins bekannt, in welchem einzelne Nadeln mit einer Schraubnabe versehen, in auf einer Kreislaufbahn angeordnete Aufnahmen eingesetzt sind, die so ausgebildet sind, dass sie in einen Nabenschaft an einer Muffe bei der Entnahme hineingedreht werden können, welche Muffe mit dem Verbindungsstück des Injektionsgerätes verbindbar ist. Das Revolvermagazin ist als Einzelteil z. B. mit fünf solcher Nadeln belegt. Eine einzelne sterile Verpackung der einzelnen Nadeln, die erst mit anderen Teilen zu einer Subkutaninjektionsnadeleinheit zusammengeführt wird, ist nicht möglich. Ebenso ist eine Verwendung für handelsübliche Subkutaninjektionsnadeln mit Schutzkappe und Aufbewahrungsbehälter nicht möglich.

Ausgehend vom Stand der Technik liegt der Erfindung die Aufgabe zugrunde, ein Magazin für mindestens eine Subkutaninjektionsnadel anzugeben, aus dem diese bei Gebrauch einfach mit einem Injektionsgerät, einer Patrone in dem Gerät oder einer Spritze verbindbar ist, wobei die Subkutaninjektionsnadeln als Einzelnadel in Aufbewahrungsbehältern verpackt sind und das Magazin auf einfache Weise bestückbar sein soll und auch eine sterile Verpackung der einzelnen Subkutaninjektionsnadeln sichergestellt ist.

Gelöst wird die Aufgabe durch Ausgestaltung des Magazins gemäß der technischen Lehre im Anspruch 1, wonach das Magazin mindestens ein Aufnahmefach zur Aufnahme mindestens eines Aufbewahrungsbehälters einer Subkutaninjektionsnadel aufweist, welches Aufnahmefach derart ausgebildet ist, dass der Aufbewahrungsbehälter senkrecht zur Längsachse des Magazins oder in einem definierten Winkel zwischen 90° und 30° verläuft und dass das Magazin an einem Gehäuse, einem Schaft oder einer Schutzkappe eines Injektionsgerätes oder an einer Spritze befestigbar ausgeführt ist oder an einem der Teile angeformt ist, wobei die Abdeckung außenseitig abziehbar zugänglich ist und das Verbindungsstück vor der Entnahme der Subkutaninjektionsnadel aus dem Aufbewahrungsbehälter mit der Muffe verbindbar ist.

Gemäß einer alternativen Ausführung ist das Magazin als befestigbare Baugruppe, z. B. als ansteck- oder anklippbares Magazin ausgebildet, das beispielsweise an die Schutzkappe eines zylinderförmigen Injektionsgerätes angesteckt oder angeschraubt werden kann oder auch seitlich auf ein Gehäuse mit einer rechteckigen Form, in welcher eine Insulinpatrone oder eine Ampulle einlegbar ist, deren Verbindungsstück aus dem Gehäuse vorsteht, klemmend aufschiebbar ist. Die Aufschiebeseiten sind dabei als Klemmseiten ausgebildet oder weisen Federelemente auf, die als Klemmelemente auf das Gehäuse aufgreifen. Im Falle der Ausbildung eines runden Gehäuses bzw. einer runden Schutzkappe eines Injektionsgerätes weist das Magazin federnde Schenkel auf, mit denen das Magazin auf die Schutzkappe seitlich aufgesteckt werden kann. Durch die Federwirkung der Schenkel, die die Schutzkappe um mindestens 180° übergreifen, ist ein sicherer Halt gegeben. Die Schenkel können auch so lang ausgebildet sein, dass sie miteinander verschraubt oder verrastet werden können. Hierfür sind bekannte Schellentechniken einsetzbar.

Im Falle, dass das Magazin auf eine Schutzkappe aufgesteckt ist, bildet es mit dieser eine von dem übrigen Teil des Injektionsgerätes abziehbare Einheit, so dass das Injektionsgerät mit dem Verbindungsstück in eine Muffe einer Nadel in dem Magazin eingesetzt werden kann. Ist die Muffe mit einem Innengewinde versehen und das Verbindungsstück mit einem Außengewinde, erfolgt ein Einschrauben, während sich die Subkutaninjektionsnadel in dem Aufbewahrungsfach befindet und wird danach aus dem Aufbewahrungsbehälter oder dem Fach herausgezogen. Zuvor muss die Versiegelungsfolie in bekannter Weise abgezogen werden, damit der Zugang der Muffe ermöglicht wird. Beim Einschrauben oder auch Einschieben durchdringt das in der Muffe sich befindende Hohlnadelende die Gummimembran in dem Verbindungsstück an der Insulinpatrone. Hier steht die Insulinpatrone für jede Art von Behältern, in denen Injektionsflüssigkeiten steril zur Verfügung gestellt werden. So können auch Ampullen zur Anwendung kommen, die eine andere Form aufweisen als normale runde Patronen, die in der Regel eine Zylinderform aufweisen und aus Glas gefertigt sind.

Die zweite Variante besteht darin, dass ein Teil des Gehäuses oder die Schutzkappe mit dem Magazin einteilig hergestellt ist, z. B. aus Kunststoff im Spritzgießverfahren, so dass die Kombination eine Einheit bildet. Hier brauchen dann am Magazin keine Klemmvorrichtungen oder Klemmseiten oder Klemmelemente vorgesehen sein, da das Magazin mit dem Gehäuseteil selbst aus Kunststoff hergestellt ist. Sollte die Schutzkappe aus Metall, z. B. Aluminium, gefertigt sein, so ist es auch möglich, dieses Teil in einem Werkzeug mit Kunststoff zu umspritzen und hierüber das Magazin anzuformen.

Gleich, welche Bauart gewählt wird, stellt sie immer den Zugang zu jeder einzelnen Subkutaninjektionsnadel, die in einem Aufbewahrungsbehälter des Magazins gelagert ist, sicher. Die Aufnahmefächer sollten dabei der Form des Aufbewahrungsbehälters für solche Subkutaninjektionsnadeln angepasst sein, so dass diese z. B. durch Eigenklemmung der Wände des Aufnahmefaches gesichert gehalten sind und dennoch gegen neue ausgetauscht werden können. Es ist aber auch möglich, die Subkutaninjektionsnadeln direkt in ein Magazin einzubringen und dort die einzelnen Nadeln zu versiegeln, so dass insbesondere bei ansteckbarer Ausführung die Subkutaninjektionsnadeln mit dem Magazin eine Verkaufseinheit bilden und die einzelnen Fächer die Aufbewahrungsbehälter. Damit die einzelnen Subkutaninjektionsnadeln, die in dem Magazin in Reihe angeordnet sind, zugänglich sind, sind die Aufnahmefächer so angeordnet, dass die Subkutaninjektionsnadeln um 90° zur Längsachse des Magazins verlaufen. Sie können aber auch in einem Winkel zwischen 90° und 30° verlaufend angeordnet sein. Dann allerdings ergeben sich an der Öffnungsseite der Aufnahmefächer eine sägezahnartige Anordnung der Aufbewahrungsbehälter oder der Aufnahmefächer selbst, in die die Subkutaninjektionsnadeln eingesetzt sind.

Die Erfindung hat den Vorteil, dass herkömmlich verpackte Subkutaninjektionsnadeln, die also steril in Aufbewahrungsbehältern einzeln verpackt sind, auch bei einem Magazin nach der Erfindung zum Einsatz kommen können. Es ist aber auch möglich, in Magazine direkt eingesetzte Subkutaninjektionsnadeln in solchen Verkaufseinheiten anzubieten. Beispielsweise können vier, acht oder zehn solcher Subkutaninjektionsnadeln in dem Magazin angeordnet sein und können auf einfache Weise durch direkte Verbindung mit dem Verbindungsstück einer Spritze oder eines Injektionsgerätes oder einer Injektionsmittelpatrone oder Ampulle direkt verbunden werden, ohne dass eine gesonderte Aufbewahrung und Handhabung erforderlich ist.

Vorteilhafte Weiterbildungen der Erfindung sind in den Unteransprüchen im Detail angegeben. Das Magazin ist preiswert aus Kunststoff herstellbar. Es kann einen sehr einfachen Aufbau aufweisen und beispielsweise aus einem U-förmig geformten Körper bestehen, in dessen Brückenteil mindestens eine Aufnahmebohrung, vorzugsweise aber eine Vielzahl von Bohrungen, eingebracht ist, in die die Aufbewahrungsbehälter der einzelnen Subkutaninjektionsnadeln einsteckbar sind. Die Dimensionierung ist dabei so aufeinander abgestimmt, dass die Aufbewahrungsbehälter klemmend in der Bohrung gehalten sind, ihr Rand aber so weit vorsteht, dass sie nach Entnahme der Subkutaninjektionsnadeln erfasst und aus der Bohrung herausgezogen werden können. Dies ist immer dann erforderlich, wenn eine Subkutaninjektionsnadel auf ein Verbindungsstück aufgesetzt worden ist und im Austausch eine neue Nadel mit Aufbewahrungsbehälter eingesetzt werden soll.

Die Seitenschenkel des U-förmigen Körpers können als federnde Klemmschenkel ausgebildet, z. B. leicht gebogen ausgeführt sein, so dass sie auf eine runde Schutzkappe des zylinderförmigen Injektionsgerätes seitlich aufgesteckt werden können und durch die Formgebung hieran klemmend gehalten werden. Ein Aufsetzen auf ein andersartig gestaltetes Gehäuse ist bei entsprechend angepasster Ausbildung der Klemmschenkel ebenfalls möglich. Auch kann eine Klemmung auf den Schaft erfolgen, also auf jenen Teil, in dem sich die Injektionsflüssigkeitspatrone oder Ampulle befindet. Auch ist ein direktes Aufstecken auf eine Spritze mit einem entsprechenden Behältnisabschnitt möglich.

Wenn das Magazin mehrere Reihen von Aufnahmefächern oder Bohrungen zur Aufnahme von den Subkutaninjektionsnadeln oder deren Aufbewahrungsbehältern aufweist, so können diese in gleicher Weise angeordnet werden. Das Magazin wird dann durch entsprechende Oberflächenwände vergrößert, in denen dann die einzelnen Fächer oder Öffnungen vorgesehen sind, um die Schutzkappen und/oder die Aufbewahrungsbehälter mit der Muffe aufnehmen zu können. Wird ein solches Magazin als Ansteckmagazin ausgeführt, so können beispielsweise an der äußeren Brückenmantelfläche zwei solcher Reihen verlaufen, die in einem definierten Radius gegenüber der Längsachse der Schutzkappe des Injektionsgerätes angeordnet sind.

Die einzelnen Aufnahmefächer oder Bohrungen können so angeordnet sein, dass die Aufbewahrungsbehälter oder die eingesetzten Subkutaninjektionsnadeln radial oder tangential zur Längsachse des Injektionsgerätes oder der Spritze verlaufend in dem Magazin angeordnet sind. In jedem Fall ist die Öffnungsseite der Muffe, die versiegelt ist, zugänglich, so dass nach Abzug der Versiegelungsfolie das Verbindungsstück der Spritze mit der Muffe verbindbar ist.

Wenn die Aufnahmen als Aufnahmefächer ausgebildet sind, ist es zweckmäßig, die Wände des Aufnahmefaches für die Subkutaninjektionsnadel oder deren Aufbewahrungsbehälter so auszubilden, dass ein Klemmdruck auf diese ausgeübt wird, so dass nur durch erhöhten Kraftaufwand die einzelnen Aufbewahrungsbehälter wieder aus dem Aufnahmefach herausziehbar sind bzw. die Subkutaninjektionsnadel, wenn sie steril in diesem Behälter gelagert ist.

Die Erfindung wird nachfolgend anhand der in den Zeichnungen dargestellten Ausführungsbeispiele ergänzend erläutert.

In den Zeichnungen zeigen:
- FIG 1: in einer isometrischen Darstellung ein Magazin nach der Erfindung zur Aufnahme von vier Subkutaninjektionsnadeln,
- FIG 2: das in FIG 1 dargestellte Ausführungsbeispiel in der Draufsicht,
- FIG 3: das in FIG 1 und in FIG 2 dargestellte Ausführungsbeispiel in der Seitenansicht,
- FIG 4: das in den FIG 1, 2 und 3 dargestellte Ausführungsbeispiel in der Stirnseitendraufsicht,
- FIG 5: ein Ausführungsbeispiel der Befestigung eines Magazins gemäß FIG 1 bis 4 an einer Schutzkappe eines Injektionsgerätes in isometrischer Darstellung und
- FIG 6: ein Beispiel eines anderen Injektionsgerätes, an dem ein Magazin ebenfalls anfügbar ist.

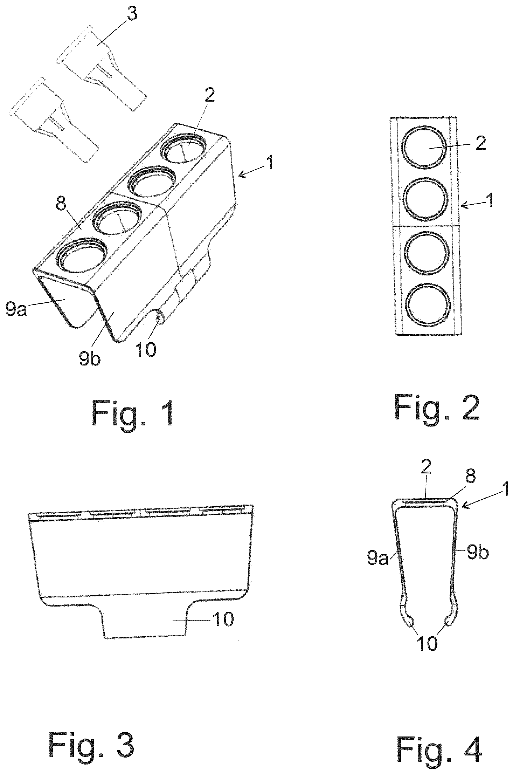

In FIG 1 ist ein Magazin 1 nach der Erfindung beispielhaft dargestellt. Dieses Magazin 1 besteht, wie die isometrische Darstellung in der FIG 1 zeigt, aus einem U-förmigen Formkörper aus Kunststoff, der im Wesentlichen aus einem Brückenteil 8 und Seitenschenkeln 9a, 9b gebildet ist. In dem Brückenteil 8 sind in Reihe vier Aufnahmebohrungen 2 eingebracht, die je ein Aufnahmefach für einen Aufbewahrungsbehälter 3 einer Subkutaninjektionsnadel bilden. Der Durchmesser der Aufnahmebohrung 2 ist dem Durchmesser des Muffenüberzugteils des Aufbewahrungsbehälters angepasst, der einen Verstärkungsring aufweist, um eine Versiegelungsfolie besser anschweißen zu können, die zur sterilen Verpackung über die Öffnung der Muffe der Nadel gelegt ist. Jede dieser einzelnen Subkutaninjektionsnadeln lässt sich mit dem Aufbewahrungsbehälter 3 in eine der Aufnahmebohrungen 2 hineindrücken und wird dort durch entsprechende Dimensionierung selbstklemmend gehalten, so dass nach Entfernen der nicht dargestellten Versiegelungsfolie am äußeren Ring des Aufbewahrungsbehälters 3 das Verbindungsstück an der Patrone oder Ampulle für das Injektionsmittel einsetzbar oder einschraubbar ist. Dies hängt von der jeweiligen Ausführung des Verbindungstückes ab, das in der Regel an einer Patrone, z. B. Insulinpatrone, vorne vorstehend angebracht ist und aus dem Injektionsgerät vorsteht. Wenn dieses Verbindungsstück eingesetzt ist, kann die Subkutaninjektionsnadel aus dem Aufbewahrungsbehälter 3 herausgezogen werden. Die Klemmkraft, die die Aufnahmebohrung 2 auf den Hülsenabschnitt des Ausbewahrungsbehälters 3 ausübt, ist dabei größer als Klemmkraft, die der Aufbewahrungsbehälter 3 auf die Muffe der Subkutaninjektionsnadel ausübt. Im Beispiel sind zwei Aufbewahrungsbehälter 3 solcher Subkutaninjektionsnadeln angedeutet eingezeichnet, obgleich vier in das Magazin hineingesteckt werden können.

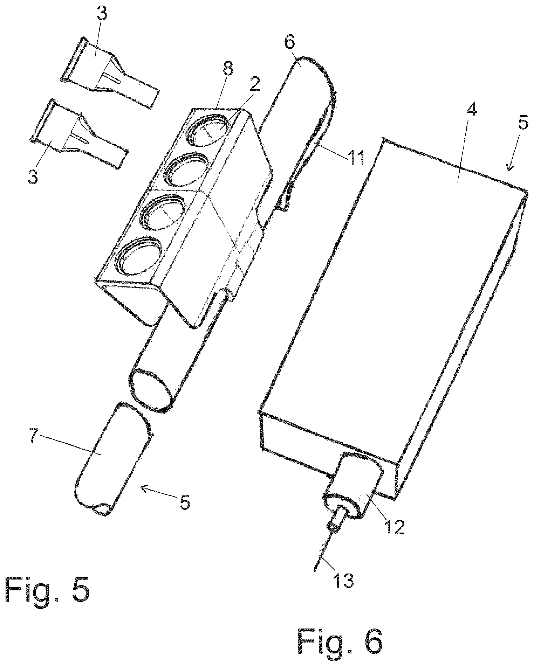

Die isometrische Darstellung zeigt ferner, dass an den Seitenschenkeln 9a und 9b mittig Klemmelemente 10 angeformt sind, die z. B. eine runde Schutzkappe 6, wie aus FIG 5 ersichtlich, teilweise klemmend umfassen. Dabei werden beim seitlichen Aufschieben die Seitenschenkel 9a und 9b nach außen gedrückt und über ihre federnde Auslegung wird ein definierter Anlagedruck ausgeübt, der größer ist als die Abzugskraft, die erforderlich ist, um eine Subkutaninjektionsnadel aus dem Aufbewahrungsbehälter 3 herausziehen zu können, wenn diese im Magazin eingesetzt ist. Das Klemmelement 10, das beidseitig an den Schenkeln 9a, 9b zusammenwirkend vorgesehen ist, umgreift einen Teil der Oberfläche einer zylinderförmigen Schutzkappe 6, wie aus FIG 5 ersichtlich, und wird daran gesichert gehalten. Die Aufbewahrungsbehälter 3 sind in die Aufnahmebohrungen 2 so eingesetzt, dass sich die nicht sichtbaren Subkutaninjektionsnadeln radial zur Längsachse der Schutzkappe 6 erstrecken.

An der Schutzkappe 6 ist ein Federklemmhalter 11 angebracht, mit dem die Schutzkappe mit dem eingesetzten, nicht vollständig dargestellten Schaft 7 des Injektionsgerätes 5 z. B. in eine Jackentasche gesteckt und an der Stoffeinfassung gesichert gehalten werden kann. Die Klemmelemente 10 können selbstverständlich auch so ausgebildet sein, dass das Magazin 1 beispielsweise auf ein quaderförmiges Injektionsgerät, wie es in FIG 6 dargestellt ist, über eine Stirnseite aufgesteckt werden kann. Dieses Injektionsgerät 5 weist ein in etwa quaderförmiges Gehäuse 4 auf. Die Betätigungsmittel zum Dosieren der Abgabemenge von Injektionsflüssigkeit, z. B. Insulin, sind nicht dargestellt. Es ist aber ersichtlich, dass auf ein vorstehendes Schraubverbindungsstück einer eingesetzten Patrone eine Muffe 12 aufgeschraubt ist. Aus Fig. 6 ist auch ersichtlich, dass hierzu das Verbindungsstück nur in eine Muffe eingeschraubt werden muss, wenn die Subkutaninjektionsnadel mit dem Aufbewahrungsbehälter in der Aufnahmebohrung 2 gelagert ist. Dazu muss allerdings das Magazin, dass klemmend an einer Seitenstirnwand das Gehäuse 4 übergreift, abgezogen werden und wird nach Entnahme einer Subkutaninjektionsnadel wieder hiermit verbunden. Die Beispiele zeigen, dass eine Schutzkappe 6 auch direkt einteilig mit einem Magazin z. B. aus Kunststoff gefertigt sein kann, wenn dies gewünscht ist. Das Gerät und die nicht eingezeichnete Subkutaninjektionsnadeln werden durch die Schutzkappe 6 nach dem Einsetzen geschützt.

### Bezugszeichenliste

- 1: Magazin
- 2: Aufnahmefach / Aufnahmebohrung
- 3: Aufbewahrungsbehälter
- 4: Gehäuse
- 5: Injektionsgerät
- 6: Schutzkappe
- 7: Schaft
- 8: Brückenteil
- 9a: Seitenschenkel
- 9b: Seitenschenkel
- 10: Klemmelement
- 11: Federklemmhalter
- 12: Muffe
- 13: Subkutaninjektionsnadel

## Patentansprüche

1. Magazin für mindestens eine Subkutaninjektionsnadel, bestehend aus einer Hohlnadel, die in einer Nadelnabe beidseitig vorstehend fixiert ist, wobei das eine Ende mit der Nadelnabe aus der Stirnwand einer auf ein Verbindungsstück einer Spritze oder eines Injektionsgerätes aufschraubbaren oder aufsteckbaren Muffe vorsteht, die das andere Ende der Hohlnadel umgibt, wobei das andere Ende bei der Fixierung der Muffe an dem Verbindungsstück eine Gummimembran, die zumindest einen Teil der Endfläche des Verbindungsstückes bildet, durchstößt, wobei die Subkutaninjektionsnadel in bekannter Weise von einem Aufbewahrungsbehälter umgeben ist, der über eine das überstehende Ende der Hohlnadel schützende Schutzkappe und die Muffe oder über die Nadel und die Muffe greift und mit einer Abdeckung an der offenen Muffenseite steril versiegelt ist, **dadurch gekennzeichnet, dass** das Magazin (1) mindestens ein Aufnahmefach (2) zur Aufnahme mindestens eines Aufbewahrungsbehälters (3) einer Subkutaninjektionsnadel aufweist, welches Aufnahmefach (2) derart ausgebildet ist, dass der Aufbewahrungsbehälter (3) senkrecht zur Längsachse des Magazins (1) oder in einem definierten Winkel zwischen 90° und 30° verläuft und dass das Magazin (1) an einem Gehäuse (4), einem Schaft (7) oder einer Schutzkappe (6) eines Injektionsgerätes (5) oder an eine Spritze befestigar ausgeführt ist, wobei die Abdeckung außenseitig abziehbar zugänglich ist und das Verbindungsstück vor der Entnahme der Subkutaninjektionsnadel aus dem Aufbewahrungsbehälter mit der Muffe verbindbar ist, wobei das Magazin (1) aus einem U-förmig geformten Formkörper besteht, in dessen Brückenteil (8) mindestens eine Aufnahmebohrung (2) zur klemmenden Lagerung des Aufbewahrungsbehälters (3) eingebracht ist, und dass die Seitenschenkel (9a, 9b) des U-förmigen Körpers als federnde Klemmschenkel ausgebildet sind, die auf ein Gehäuse (4) oder einen Schaft oder einer Schutzkappe (6) eines Injektionsgerätes (5) aufschiebbar, aufsteckbar oder anklippbar sind, wobei die Längsachse des Magazins parallel zur Längsachse des Injektionsgerätes verläuft.

2. Magazin nach Anspruch 1, **dadurch gekennzeichnet, dass** das Magazin (1) aus Kunststoff besteht.

3. Magazin nach Anspruch 1 und 2, **dadurch gekennzeichnet, dass** das Magazin (1) und die Schutzkappe (6) des Injektionsgerätes einteilig aus Kunststoff gefertigt sind.

4. Magazin nach Anspruch 1, **dadurch gekennzeichnet, dass** das mindestens eine Aufnahmefach (2) oder die mindestens eine Aufnahmebohrung (2) oder mehrere Aufnahmefächer in Reihe radial oder tangential zur Längsachse des Injektionsgerätes oder der Spritze verlaufend in dem Magazin angeordnet sind.

5. Magazin nach Anspruch 1, **dadurch gekennzeichnet, dass** die Aufnahmefächer (2) eine Lagerkammer aufweisen, die der Form des Aufnahmebehälters angepasst ist.

6. Magazin nach Anspruch 5, **dadurch gekennzeichnet, dass** in dem Aufnahmefach (2) Klemmelemente zur lösbaren Fixierung des Aufbewahrungsbehältnisses angeordnet sind.

7. Magazin nach Anspruch 1, **dadurch gekennzeichnet, dass** die Aufnahmefächer (2) zugleich die Aufbewahrungsbehälter für einzelne Subkutaninjektionsnadel sind und diese direkt in die Aufnahmefächer eingesetzt und steril verschlossen sind.

8. Magazin nach Anspruch 1, **dadurch gekennzeichnet, dass** mindestens eine Reihe von Aufnahmefächern in dem Magazin (1) zur Aufnahme mehrerer Subkutaninjektionsnadeln oder deren Aufbewahrungsbehälter (3) vorgesehen ist.

9. Magazin nach Anspruch 8, **dadurch gekennzeichnet, dass** mehrere Reihen von Aufnahmefächern (2) im Magazin (1) vorgesehen sind und dass diese in den das Gehäuse überstehenden Wänden angeordnet sind.

## Claims

1. Cartridge for at least one subcutaneous injection needle, consisting a hollow needle fixed in a needle hub to protrude at both ends, wherein one end protrudes together with the needle hub from the end wall of a sleeve which is able to be screwed or plugged onto a connecting member of a syringe or injection apparatus and which surrounds the other end of the hollow needle, wherein the other end during fixing of the sleeve to the connecting member penetrates a rubber membrane forming at least a part of the end surface of the connecting member, and wherein the subcutaneous injection needle in known manner is surrounded by a storage container which engages over a protective cap protecting the protruding end of the hollow needle and over the sleeve or over the needle and the sleeve and is sealed in sterile manner by a cover at the open sleeve end, **characterised in that** the cartridge (1) has at least one receiving compartment (2) for reception of at least one storage container (3) of a subcutaneous injection needle, which receiving compartment (2) is so constructed that the storage container (3) extends perpendicularly to the longitudinal axis of the cartridge (1) or at a defined angle between 90° and 30° and that the cartridge (1) is constructed to be securable to a housing (4), a shank (7) or a protective cap (6) of injection apparatus (5) or to a syringe, wherein the cover is externally accessible for pulling off and the connecting member is connectible with the sleeve before removal of the subcutaneous injection needle from the storage container, wherein the cartridge (1) consists of a moulded body of U-shaped form, in the bridge part (8) of which at least one receiving bore (2) for mounting of the storage container (3) by clamping is formed, and that the lateral limbs (9a, 9b) of the U-shaped body are constructed as resilient clamping limbs which can be pushed, plugged or clipped onto a housing (4) or a shank or a protective cap (6) of injection apparatus (5), wherein the longitudinal axis of the cartridge extends parallel to the longitudinal axis of the injection apparatus.

2. Cartridge according to claim 1, **characterised in that** the cartridge (1) consists of plastics material.

3. Cartridge according to claim 1 and 2, **characterised in that** the cartridge (1) and the protective cap (6) of the injection apparatus are made integrally from plastics material.

4. Cartridge according to claim 1, **characterised in that** the at least one receiving compartment (2) or the at least one receiving bore (2) or several receiving compartments is or are arranged in a cartridge in a row to extend radially or tangentially to the longitudinal axis of the injection apparatus or the syringe.

5. Cartridge according to claim 1, **characterised in that** the receiving compartments (2) comprise a storage chamber adapted to the shape of the receiving container.

6. Cartridge according to claim 5, **characterised in that** clamping elements for releasable fixing of the storage container are arranged in the receiving compartment (2).

7. Cartridge according to claim 1, **characterised in that** the receiving compartments (2) are at the same time the storage container for individual subcutaneous injection needles and these are directly inserted into the receiving compartments and closed in sterile manner.

8. Cartridge according to claim 1, **characterised in that** at least one row of receiving compartments is provided in the cartridge (1) for reception of several subcutaneous injection needles or the storage containers (3) thereof.

9. Cartridge according to claim 8, **characterised in that** several rows of receiving compartments (2) are provided in the cartridge (1) and that these are arranged in the walls projecting beyond the housing.

## Revendications

1. Magasin pour au moins une aiguille d'injection sous-cutanée, constituée d'une aiguille creuse, qui est fixée de manière à faire saillie bilatéralement dans un pavillon d'aiguille, dans lequel l'une des extrémités, comportant le pavillon de l'aiguille, fait saillie de la paroi frontale d'un manchon pouvant être vissé ou enfiché sur une pièce de liaison d'une seringue ou d'un appareil d'injection, lequel manchon entoure l'autre extrémité de l'aiguille creuse, dans lequel l'autre extrémité, lors de la fixation du manchon sur la pièce de liaison, perce une membrane en caoutchouc, qui forme au moins une partie de la surface d'extrémité de la pièce de liaison, dans lequel l'aiguille d'injection sous-cutanée est entourée, d'une manière connue, par un réservoir de stockage qui chevauche un capuchon protecteur protégeant l'extrémité en saillie de l'aiguille creuse et le manchon, ou l'aiguille et le manchon, et est scellée de façon stérile par un cache au niveau de la face ouverte du manchon, **caractérisé en ce que** le magasin (1) comprend au moins un compartiment de réception (2) pour la réception d'au moins un réservoir de stockage (3) d'une aiguille d'injection sous-cutanée, lequel compartiment de réception (2) est réalisé de telle manière que le réservoir de stockage (3) s'étend perpendiculairement à l'axe longitudinal du magasin (1) ou selon un angle défini compris entre 90° et 30° et **en ce que** le magasin (1) est conçu de manière à pouvoir être fixé sur un boîtier (4), une tige (7) ou un capuchon protecteur (6) d'un appareil d'injection (5) ou sur une seringue, dans lequel le cache est accessible de l'extérieur et peut être extrait et la pièce de liaison, avant que l'aiguille d'injection sous-cutanée soit retirée du réservoir de stockage, peut être reliée au manchon, dans lequel le magasin (1) est constitué d'un corps moulé en forme de U, dans la partie formant pont (8) duquel est ménagé au moins un trou de réception (2) pour le montage avec serrage du réservoir de stockage (3), et **en ce que** les branches latérales (9a, 9b) du corps en forme de U sont réalisées sous la forme de branches de serrage élastiques, qui peuvent être enfilées, enfichées ou clipsées sur un boîtier (4) ou une tige ou un capuchon protecteur (6) d'un appareil d'injection (5), dans lequel l'axe longitudinal du magasin s'étend parallèlement à l'axe longitudinal de l'appareil d'injection.

2. Magasin selon la revendication 1, **caractérisé en ce que** le magasin (1) est constitué de matière plastique.

3. Magasin selon les revendications 1 et 2, **caractérisé en ce que** le magasin (1) et le capuchon protecteur (6) de l'appareil d'injection sont fabriqués d'une seule pièce en matière plastique.

4. Magasin selon la revendication 1, **caractérisé en ce que** ledit au moins un compartiment de réception (2) ou ledit au moins un trou de réception (2) ou plusieurs compartiments de réception sont agencés dans le magasin de manière à s'étendre en série de manière radiale ou tangentielle par rapport à l'axe longitudinal de l'appareil d'injection ou de la seringue.

5. Magasin selon la revendication 1, **caractérisé en ce que** les compartiments de réception (2) comprennent une chambre de stockage qui est adaptée à la forme du réservoir de réception.

6. Magasin selon la revendication 5, **caractérisé en ce que** des éléments de serrage permettant la fixation libérable du réservoir de stockage sont agencés dans le compartiment de réception (2).

7. Magasin selon la revendication 1, **caractérisé en ce que** les compartiments de réception (2) sont également les réservoirs de stockage pour différentes aiguilles d'injection sous-cutanée et celles-ci sont insérées directement dans les compartiments de réception et fermées de manière stérile.

8. Magasin selon la revendication 1, **caractérisé en ce qu'**au moins une rangée de compartiments de réception est prévue dans le magasin (1) pour recevoir plusieurs aiguilles d'injection sous-cutanée ou les réservoirs de stockage (3) de celles-ci.

9. Magasin selon la revendication 8, **caractérisé en ce que** plusieurs rangées de compartiments de réception (2) sont prévues dans le magasin (1) et **en ce que** celles-ci sont agencées dans les parois faisant saillie du boîtier.
